# EUROPEAN PATENT SPECIFICATION

(11) **EP 1 155 660 B1**
(45) Date of publication and mention of the grant of the patent: **10.12.2003**
(21) Application number: 00830349.7
(22) Date of filing: 15.05.2000
(51) Int. Cl.: A61B 17/12

(54) **Haemorrhoidal ligature applicator**
Instrument zum Abbinden von Hämorrhoiden
Instrument pour la ligature des hémorrhoides

(43) Date of publication of application: 21.11.2001
(73) Proprietor: Sapi Med S.p.A., 15100 Alessandria (IT)
(72) Inventor: Oddenino, Gian Paolo, 15100 Alessandria (IT)
(74) Representative: Long, Giorgio

(56) References cited:
- EP-A- 0 502 477
- DE-A- 19 834 263
- DE-U- 9 205 453
- DE-U- 29 823 317
- US-A- 3 382 873
- US-A- 3 989 049
- US-A- 4 257 419

## Description

The present invention relates to a medical device for the application of resilient ligatures to haemorrhoids, commonly known as a haemorrhoidal ligature applicator.

It is known that haemorrhoids are constituted by small venous swellings, so-called piles, which may even emerge from the anus and cause the patient pain, pruritis and even bleeding. The most recent surgical treatment for haemorrhoids is based on the application of a resilient means to the haemorrhoid so that the vein is closed at the base and then, since the haemorrhoid is no longer supplied with blood, it tends to become sclerosed and to fall off spontaneously. This operation is easier and quicker than the conventional operation and can also be carried out almost painlessly and as an outpatient operation, by giving the patient a local anaesthetic. This operation, which is known as "tying" the haemorrhoids, is carried out by means of a suitable ligature applicator constituted basically by a pistol the barrel of which is connected to suction means. A cylinder arranged coaxially and slidably outside the barrel is operated by a trigger. The slidable cylinder terminates slightly before the distal end of the barrel, thus leaving a portion of the barrel free. Suitable resilient means for tying the haemorrhoids are fitted on this free portion. Clearly, for reasons of hygiene and convenience of use, these ligature applicators must be disposable. It is therefore extremely important for them to have considerable structural simplicity such as to minimize their production cost, and to be made of inexpensive materials.

A further problem which has been found in relation to the haemorrhoidal ligature applicators of the prior art is that the resilient ligature rings are fitted on the applicator with considerable difficulty, in spite of the use of a suitable loading cone. This difficulty is due to the fact that the resilient rings have to be force-fitted onto the ligature applicator so that, once they are fitted around the haemorrhoid, they can exert an adequate constricting force to achieve the desired result.

EP-A-502477 discloses a haemorrhoid ligation instrument generally as in claim 1, but with a loading cone according to the state of the art. US-A-4257419, upon which disclosure the two part form of claim 17 is based and which represents the closest prior art regarding the loading cone of claim 1, discloses a loading cone for a haemorrhoid ligation instrument with a single step provided in the generally conical section.

The problem upon which the present invention is based is therefore that of providing a haemorrhoidal ligature applicator which overcomes the disadvantages of the prior art and which, in particular, is of simple construction and low cost.

This problem is solved by a haemorrhoidal ligature applicator as outlined in the appended claims.

Further characteristics and advantages of the haemorrhoidal ligature applicator of the present invention will become clearer from the description of some preferred embodiments thereof, given below by way of non-limiting example, with reference to the following drawings:
Figure 1 is a perspective view of a haemorrhoidal ligature applicator according to the present invention,
Figure 2 is a perspective view of a detail of an alternative haemorrhoidal ligature applicator according to the present invention,

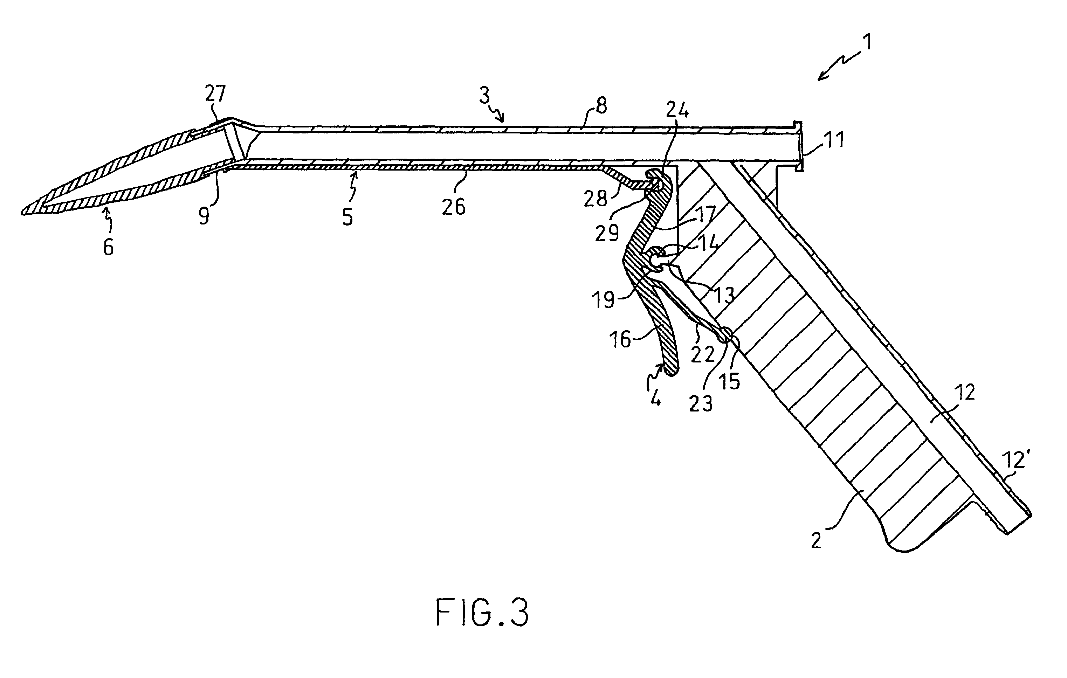

Figure 3 is a side view showing the haemorrhoidal ligature applicator of Figure 1 in section,
Figure 4 is a perspective view showing the detail of the trigger of the haemorrhoidal ligature applicator according to the present invention,
Figure 5 is a perspective view showing the detail of the slidable actuator of the haemorrhoidal ligature applicator according to the present invention,
Figure 6 is a side view showing the detail of the loading cone and the distal end of the haemorrhoidal ligature applicator according to the present invention, in section,
Figure 7 is a perspective view of the haemorrhoidal ligature applicator according to the present invention in the condition of use.

With reference to the drawings, the haemorrhoidal ligature applicator according to the present invention is generally indicated 1. The ligature applicator comprises, basically, a grip 2 fixed to a barrel 3. The applicator also comprises a trigger 4 which acts on a slidable actuator 5. Finally, the haemorrhoidal ligature applicator 1 is completed by a cone 6 for loading the resilient rings 7.

The barrel 3 is composed of a tubular body 8 the cylindrical distal end 9 of which is inclined downwardly relative to the longitudinal axis of the tubular body itself and terminates in an opening 10. The inclination of the distal end 9 is preferably 18°.

A second, vacuum-relief opening 11 is disposed at the opposite end of the tubular body 8.

The grip 2 extends along an axis inclined downwardly relative to the longitudinal axis of the tubular body 8 and comprises a vacuum duct 12 which opens into the inner portion of the tubular body 8 in the region of the point of attachment of the grip 2 to the barrel 3. The free end of the duct 12 has a ridged connector 12' for the fitting of a resilient vacuum tube.

The grip 2 and the barrel 3 are preferably formed integrally.

On the front of the grip 2, there is a pin 13 terminating in a cylindrical appendage 14 having a bulbous cross-section. Below the pin 13, the grip 2 also has a recess 15 the inner surface of which is semicylindrical.

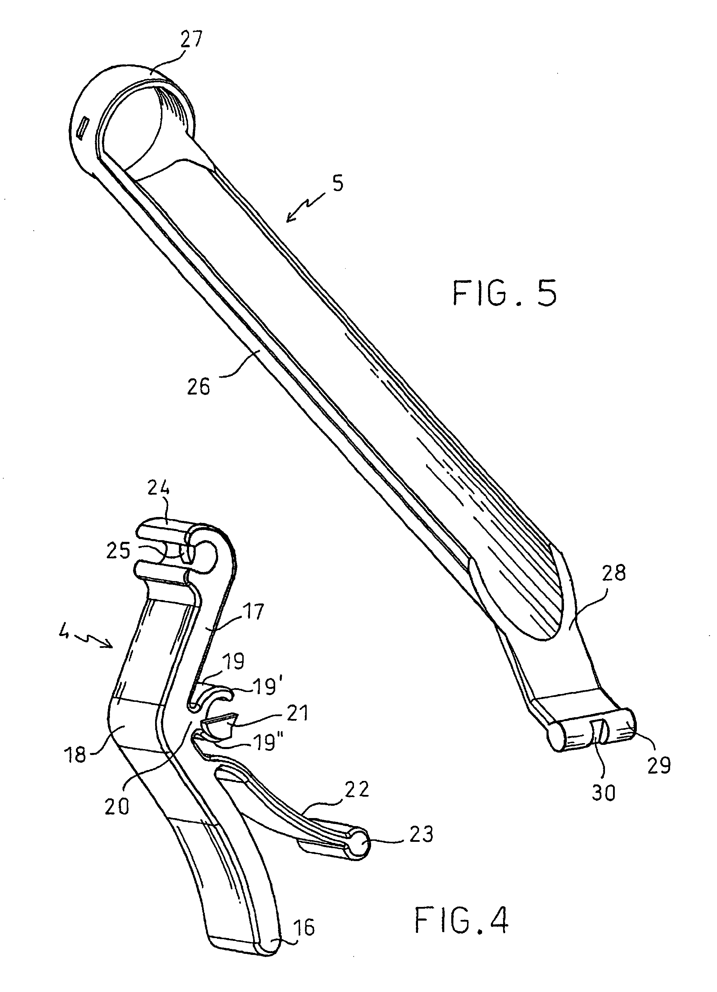

With particular reference to Figure 4, the trigger 4 comprises a first arm 16 and a second arm 17 which are connected by a central portion 18 on which the trigger is mounted for pivoting. For this purpose, the central portion 18 has a projecting seat 19. This seat 19 is intended to house the pin 13 of the grip 2 by snap-engaging the appendage 14 of the pin. The seat 19 is thus constituted by two claws 19', 19'' with semicylindrical inner surfaces which extend from a stalk 20 connected to the central portion 18 of the trigger; the seat 19 therefore has a shape complementary to that of the appendage 14 of the pin 13 of the grip 2.

The seat 19 is partially closed laterally by a pair of side walls 21 (only one side wall is shown in the drawings) which extend from the edges of the lower claw 19'' as far as the connecting point of the two claws. Naturally, the side walls 21 could cover only the upper halves of the sides of the seat 19, or they could cover the sides completely.

In the embodiment shown in the drawings, the arms 16, 17 of the trigger 4 do not lie on the same axis but on axes which converge in the region of the central portion 18 of the trigger, the seat 19 being included in the angle formed by the axes. It is, however, possible to form a trigger in which the two arms 16, 17 lie on the same axis.

The lower arm 16 comprises a tongue 22 on the same side on which the seat 19 is disposed. The tongue 22 extends downwards along an axis diverging from the arm 16 and has a substantially bent cross-section. The tongue 22 terminates in a cylindrical appendage 23 which has a bulbous cross-section and which is intended to engage the respective seat 15 of the grip 2. This appendage 23 therefore has a shape and a size complementary to those of the seat 15.

At the end of the upper arm 17, there is a seat 24 the function of which will become clear from the following description. In the same manner as the seat 19 described above, the seat 24 has a substantially C-shaped cross-section. In a central position on the inner surface of the seat 24 there is a tooth 25 with an arcuate edge.

With particular reference to Figure 5, the slidable actuator 5 comprises a body 26 having a semicylindrical inner surface. The body 26 terminates at one end in a ring 27 having an inside diameter substantially corresponding to the outside diameter of the distal end 9 of the barrel 3. The ring 27 is inclined to the longitudinal axis of the body 26 at substantially the same angle as the inclination of the distal end 9 to the barrel 3. This angle is preferably 18°.

At the end remote from that carrying the ring 27, the body 26 of the slidable actuator 5 has a tongue 28 terminating in a cylindrical appendage 29 with a substantially bulbous cross-section. The appendage 29 has a notch 30 in its central portion. The appendage 29 can thus snap-engage the seat 24 of the upper arm 17 of the trigger 4, the tooth 25 of which fits in the notch 30.

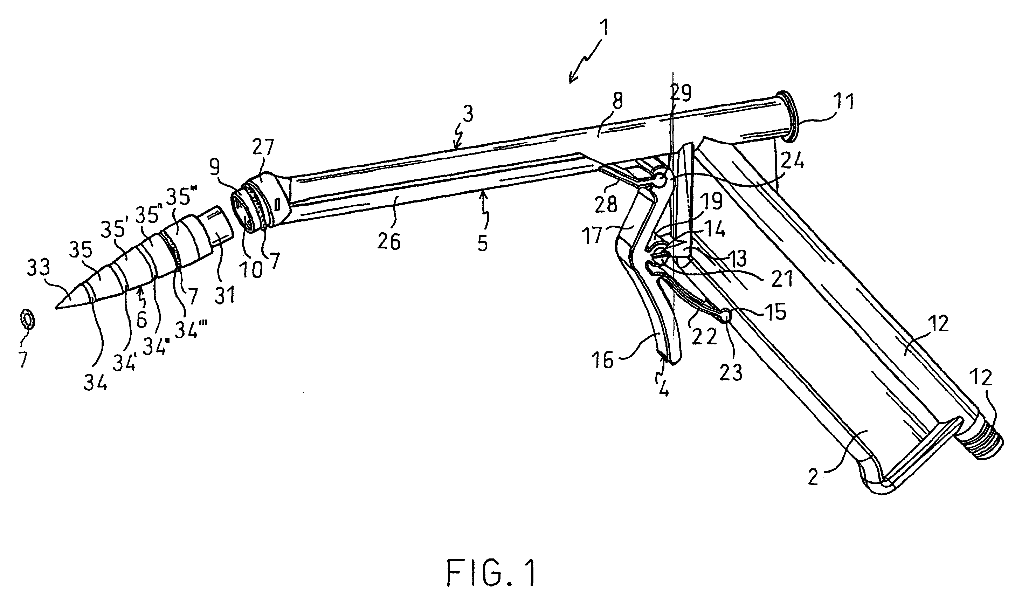

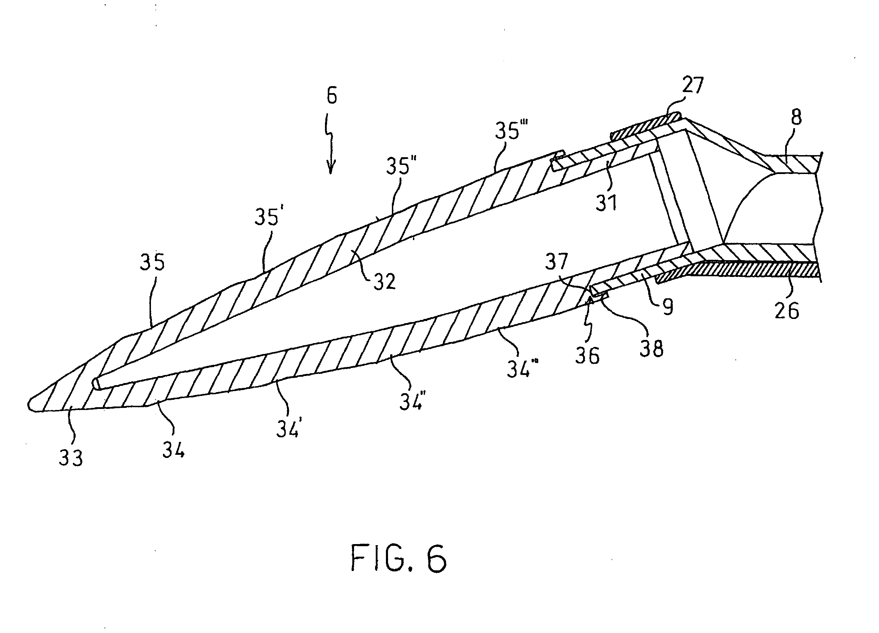

With particular reference to Figures 1 and 6, the loading cone 6 comprises a tubular connecting portion 31 and a conical portion 32. In the drawings, the loading cone 6 is shown as a hollow body, but it could just as well be solid.

The outer surface of the conical portion 32 has a conical tip surface 33 adjoining a first step 34. A plurality of frustoconical surfaces 35, 35', 35'', 35''', spaced apart by respective steps 34', 34'', 34''', adjoin the step 34.

The steps 34, 34', 34'', 34''' are constituted by cylindrical surfaces the diameters of which increase from step to step and which are arranged coaxially relative to the longitudinal axis of the loading cone 6.

The conical tip surface 33 and the successive frustoconical surfaces 35, 35', 35'', 35''' preferably have inclinations to the longitudinal axis of the loading cone 6 which decrease from one surface to the next. In a particularly preferred aspect of the present invention, the loading cone 6 comprises four frustoconical surfaces 35, 35', 35'', 35''' in addition to the conical tip surface 33. Even more preferably, these surfaces have inclinations to the longitudinal axis of the loading cone 6 of 15° (for the conical tip surface 33), 10°, 8°, 5° and 3°, respectively.

The tubular connecting portion 31 has an outside diameter substantially corresponding to the inside diameter of the distal end 9 of the barrel 3, so that the tubular portion can be fitted inside the distal end of the barrel. The joining region between the tubular connecting portion 31 and the conical portion 32 of the loading cone 6 has an annular seat 36 having a base 37 and an outer wall 38. The base 37 has a width substantially corresponding to the thickness of the distal end 9 of the barrel 3 so that the end portion of the distal end 9 is inserted in the annular seat 36. The outer wall 38 of the annular seat 36 thus covers the outer surface of the end portion of the distal end 9 of the barrel 3.

The haemorrhoidal ligature applicator according to the present invention may be made of various materials. It is however, preferably made of plastics material, particularly for reasons of cost; the haemorrhoidal ligature applicator according to the present invention is in fact preferably disposable which would render applicators made of more expensive materials definitely disadvantageous.

It is, however, important that the material used, particularly with regard to the trigger 4 and the slidable actuator 5, should have adequate resilience properties, for the reasons which will become clear from the following description of the operation of the device. Particularly preferred materials are shock-resistant polystyrene or polycarbonate.

The haemorrhoidal ligature applicator 1 is assembled in the following manner. The trigger 4 is positioned on the grip 2 by snap-engaging the appendage 14 of the pin 13 in the respective seat 19 of the trigger and housing the appendage 23 of the tongue 22 of the trigger in the respective seat 15 of the grip 2. The slidable actuator 5 is then mounted on the barrel 3 by fitting the ring 27 on the distal end 9 of the barrel and fitting the body 26 of the actuator closely against the lower surface of the tubular body 8 of the barrel. The appendage 29 of the tongue 28 of the slidable actuator 5 is then snap-engaged in the seat 24 disposed on the upper arm 17 of the trigger 4, so that the tooth 25 of the seat 24 is fitted in the notch 30 of the appendage 29. Finally, the loading cone 6 is positioned on the distal end 9 of the barrel 3.

The resilient rings 7 are then fitted on the distal end 9 of the barrel 3 in the following manner. The resilient ring is slid along the conical surface of the loading cone and is thus expanded to an ever greater extent. The steps 34, 34', 34'', 34''' represent a corresponding number of abutment points for the resilient ring; the rings can thus be loaded in steps and, should they slip out of the user's hand as a result of being stretched, they are prevented from returning to the initial position in which they are released from the cone. In fact, to give an example, if a user were to lose his grip on a resilient ring 7 which had reached the last frustoconical surface 35''', as the ring returned backwards, it would abut the immediately preceding step 34''' rather than sliding as far as the position in which it is released from the cone 6.

The resilient ring 7 is thus positioned on the outer surface of the distal end 9 of the barrel 3. However, by virtue of the presence of the outer wall 38 of the annular seat 36 on the end portion of the barrel, the resilient ring 7 is disposed a certain distance from the edge of the distal end 9. This prevents the resilient ring from slipping off the distal end 9 of the barrel 3 easily once the loading cone has been removed.

By operating as described above, it is also possible to load more than one resilient ring 7 on the distal end of the barrel 3 so as to achieve a stronger ligature of the haemorrhoid.

The resilient rings 7 are made of a suitable elastomer which should ensure considerable deformability combined with good mechanical strength and chemical characteristics. A preferred material is natural rubber.

After the resilient ring 7 has been loaded on the distal end 9 of the barrel 3, the loading cone 6 is removed. The haemorrhoidal ligature applicator is then connected to a vacuum means, for example, to a pump, by fitting the respective tube onto the connector 12' on the grip 2.

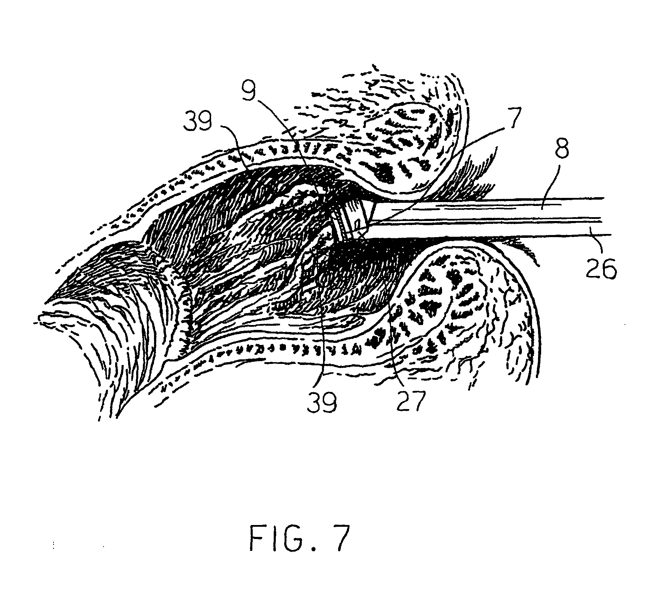

As shown in Figure 7, the device is then inserted in the patient's anus in the vicinity of a haemorrhoid 39. The instrument is gripped by the doctor with one hand, in the same manner as a pistol. The doctor's thumb can then be placed over the vacuum-relief opening 11 in the barrel 3, thus enabling the haemorrhoid 39 to be drawn into the barrel of the ligature applicator through the opening 10 in the distal end 9. At this point, by operation of the trigger, which pivots about the pin 13, the slidable actuator is advanced and acts on the resilient ring 7 until the ring is disengaged from the distal end 9 of the barrel 3; the resilient ring, which was force-fitted on the distal end of the barrel, will return to its initial shape, squeezing the base of the haemorrhoid 39 tightly. At this point, the thumb can be removed from the opening 11, thus interrupting the suction on the haemorrhoid. As stated above, the haemorrhoid, which is deprived of a blood supply, will tend to sclerose and fall off.

When the trigger is released, it returns to the starting position, also returning the slidable actuator 5 backwards. The trigger can be returned owing to the presence of the tongue 22 which acts as a spring against the wall of the grip 2. This is the reason why the material of which the trigger is made must have adequate resilience properties.

The operation is repeated on the various haemorrhoids 39 from which the patient is suffering.

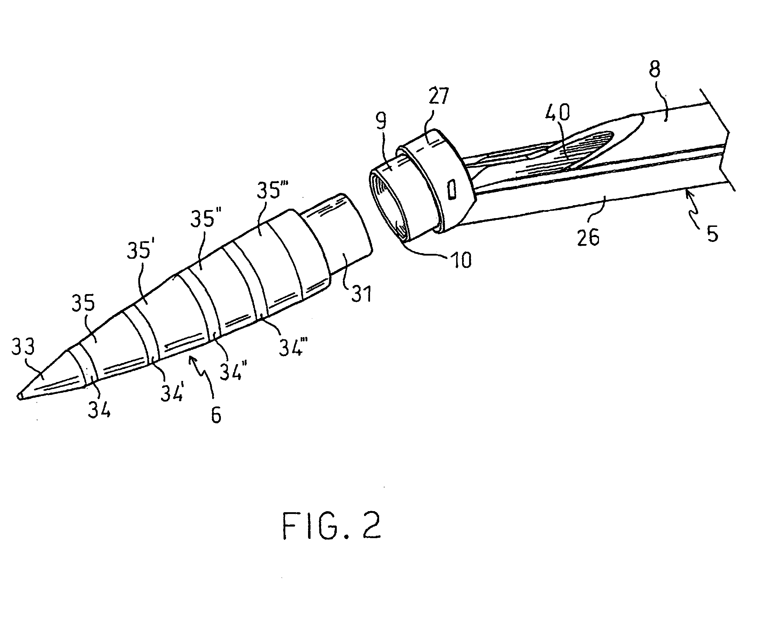

In a second embodiment of the present invention, shown in Figure 2, the tubular body 8 has a slot-like opening 40 in its upper portion, in the vicinity of the distal end 9. Surgical forceps can be introduced through this opening and, by extending through the opening 10 in the distal end 9, can grip a haemorrhoid 39 and bring it into the distal end. The ligature is then applied to the haemorrhoid in exactly the same manner as described above for the first embodiment of the invention.

In the second embodiment just described, it is not therefore necessary to provide for a connection of the ligature applicator to vacuum means. The ligature applicator may therefore not comprise the duct 12 or the opening 11 in the barrel 3, although clearly their presence in no way hinders the use of forceps.

The haemorrhoidal ligature applicator according to the invention has many advantages.

First of all, as described above, the particular shape of the loading cone 6 enables the resilient rings 7 to be loaded particularly conveniently and securely. The presence of the outer wall 28 which abuts the resilient rings sufficiently far from the edge of the distal end 9 prevents the rings from inadvertently coming off the distal end once the loading cone has been removed.

The inclination of the distal end 9 to the longitudinal axis of the barrel 3 is designed to facilitate the operation of locating the haemorrhoid, since the haemorrhoid is disposed along the anus wall, and to afford the operator an excellent view of the haemorrhoid.

The structural characteristics of the instrument have been designed to achieve maximum functionality at the lowest possible cost, which is also made possible by the preferred use of plastics material. In particular, the presence of the walls 21 on the seat 19 and of the coupling between the tooth 25 of the seat 24 and the notch 30 in the appendage 29 of the actuator keep the assembly constituted by the pin 13, the trigger 4 and the actuator 5 aligned.

The embodiment which comprises the slot-shaped opening 40 in the barrel 3 is particularly advantageous when a pump or other vacuum device is not available.

As stated, by virtue of its convenience in use, its simple construction and its low cost, the haemorrhoidal ligature applicator according to the present invention constitutes an ideal disposable instrument for applying ligatures to haemorrhoids.

Clearly, only some embodiments of the haemorrhoidal ligature applicator of the present invention have been described and an expert in the art will be able to apply thereto all of the modifications necessary for their adaptation to particular applications without, however, departing from the scope of protection of the present claims.

## Claims

1. A device for applying ligatures to haemorrhoids, comprising a barrel (3) provided with a grip (2) and having a distal end (9) which can receive resilient rings (7), a slidable actuator (5), operated by a trigger (4), being disposed on the outer surface of the barrel (3), the slidable actuator (5) being movable between a retracted position and an advanced position so as to cause the resilient ring (7) to be disengaged from the distal end (9) of the barrel (3) upon command, said device further comprising a loading cone (6) for the resilient rings (7) , the loading cone (6) being capable of being fitted removably in the opening (10) of the distal end (9) of the barrel (3) and comprising a generally conical portion (32) and a tubular connecting portion (31) which is adapted to be fitted in the opening (10) in the distal end (9) of the barrel (3), the outer surface of the conical portion (32) comprising a conical tip surface (33) and a plurality of frustoconical surfaces (35, 35', 35'', 35'''), the conical tip surface and the frustoconical surfaces being spaced apart by respective cylindrical steps (34, 34', 34'', 34''').

2. A device according to Claim 1, in which the barrel (3) is constituted basically by a tubular body (8) having a vacuum-relief opening (11) disposed at the end of the barrel (3) remote from the distal end (9), and in which the grip (2) comprises a vacuum duct (12), the tubular body (8) and the duct (12) being in fluid communication.

3. A device according to Claims 1 or 2, in which the barrel (3) has a slot-like opening (40) in its upper portion in the vicinity of the distal end (9), for the introduction of surgical forceps.

4. A device according to Claim 1, in which the conical tip surface (33) and the frustoconical surfaces (35, 35' , 35'', 35''') have respective inclinations to the longitudinal axis of the loading cone (6) which decrease in order from one surface to the next.

5. A device according to Claim 4, in which the loading cone (6) comprises four frustoconical surfaces and in which the inclinations of the conical tip surface (33) and of the frustoconical surfaces (35, 35', 35'', 35''') to the longitudinal axis of the loading cone (6) are 15°, 10°, 8°, 5°, and 3°, respectively.

6. A device according to Claim 1, in which the joining region between the conical portion (32) and the tubular connecting portion (31) of the loading cone (6) comprises an annular seat (36) having a base (37) and an outer wall (38) so that, when the loading cone (6) is fitted in the opening (10) in the distal end (9) of the barrel (3), the outer wall (38) of the annular seat (36) covers the end portion of the distal end (9).

7. A device according to any one of Claims 1 to 6, in which the distal end (9) of the barrel (3) is inclined to the longitudinal axis of the barrel (3).

8. A device according to Claim 7, in which the distal end (9) is inclined downwardly by 18°.

9. A device according to any one of Claims 1 to 8, in which the trigger (4) is articulated to the grip (2) and to the slidable actuator (5) by snap means (14, 19; 24, 29).

10. A device according to Claim 9, in which the snap means comprise cylindrical appendages (14, 29) which have bulbous cross-sections and which can be snap-engaged in complementary seats (19, 24).

11. A device according to Claim 10, in which the seat (19) is partially or completely closed laterally by a pair of side walls (21).

12. A device according to Claim 10, in which a tooth (25) with an arcuate edge is disposed in a central position on the inner surface of the seat (24), and in which the appendage (29) has a notch (30) in its central portion, the tooth (25) being intended to engage the notch (30).

13. A device according to any one of Claims 1 to 12, in which the trigger (4) comprises a tongue (22) which acts resiliently on the grip (2).

14. A device according to any one of Claims 1 to 13, in which the slidable actuator (5) comprises a body (26) having a semicylindrical inner surface and terminating at one end in a ring (27) which is intended to be engaged slidably on the distal end (9) of the barrel (3).

15. A device according to any one of Claims 1 to 14, in which the device is made of shock-resistant polystyrene or of polycarbonate.

16. A device according to any one of Claims 1 to 15 in which the device is disposable.

17. A loading cone (6) for a device for applying ligatures to haemorrhoids , said loading cone having a generally conical portion (32) linked at its base by an annular seat (36) to a tubular connecting portion (31) of diameter smaller than the diameter of the base of the generally conical portion, the outer surface of said generally conical portion comprising a conical tip surface (33) and a first frustoconical surface (35), connected to the conical tip surface by a cylindrical step surface (34), **characterised in that** the outer surface of the generally conical portion further comprises at least one further frustoconical surface (35', 35", 35''') connected respectively by further cylindrical step surfaces (34',34",34"').

18. A loading cone according to Claim 17, in which the conical tip surface (33) and the frustoconical surfaces (35, 35', 35'', 35''') have respective inclinations to the longitudinal axis of the loading cone (6) which decrease in order from one surface to the next.

19. A loading cone according to Claim 18, in which the loading cone (6) comprises four frustoconical surfaces and in which the inclinations of the conical tip surface (33) and of the frustoconical surfaces (35, 35', 35'', 35''') to the longitudinal axis of the loading cone (6) are 15°, 10°, 8°, 5°, and 3°, respectively.

20. A loading cone according to Claim 17, in which the joining region between the generally conical portion (32) and the tubular connecting portion (31) of the loading cone (6) comprises an annular seat (36) having a base (37) and an outer wall (38) so that, when the loading cone (6) is fitted in the opening (10) in the distal end (9) of the barrel (3), the outer wall (38) of the annular seat (36) covers the end portion of the distal end (9).

21. A kit comprising a device for applying ligatures to haemorrhoids according to any one of Claims 1 to 16, one or more loading cones (6) according to any one of Claims 17-20, and one or more resilient rings (7).

22. A kit according to Claim 18, in which the resilient rings (7) are made of natural rubber.

## Patentansprüche

1. Instrument zum Abbinden von Hämorrhoiden, mit einem Tubus (3), der mit einem Griff (2) versehen ist und ein distales Ende (9) aufweist, das elastische Ringe (7) aufnehmen kann, mit einem verschiebbaren Betätigungsglied (5), das von einem Abzug (4) betätigt wird, sich auf der Außenfläche des Tubus (3) befindet und zwischen einer zurück gezogenen Lage und einer vorgeschobenen Lage hin und her bewegbar ist, um den elastischen Ring (7) willkürlich vom distalen Ende (9) des Tubus (3) zu lösen, und weiterhin mit einem Ladekegel (6) für die elastischen Ringe (7), der in die Öffnung (10) am distalen Ende (9) des Tubus (3) herausnehmbar einsetzbar ist und einen allgemein konischen Teil (32) und einen rohrförmigen Verbindungsteil (31) aufweist, der in die Öffnung (10) im distalen Ende (9) des Tubus (3) einsetzbar ist, wobei die Außenfläche des konischen Teils (3) an der Spitze eine konische Fläche (33) und eine Vielzahl von kegelstumpfförmigen Flächen (35, 35', 35", 35"') aufweist und die konische Spitzenfläche und die kegelstumpfförmigen Flächen jeweils von zylindrischen Absätzen (34, 34', 34", 34"') getrennt sind.

2. Instrument nach Anspruch 1, dessen Tubus (3) im Prinzip aus einem rohrförmigen Körper (8) mit einer Unterdruck-Entlastungsöffnung (11) am vom distalen Ende (9) entgegengesetzten Ende des Tubus (3) besteht, wobei der Griff (2) einen Unterdruckkanal (12) aufweist und der rohrförmige Körper (8) mit dem Kanal (12) in Strömungsverbindung steht.

3. Instrument nach Anspruch 1 oder 2, dessen Tubus (3) in seinem oberen Bereich in der Nähe des distalen Endes (9) eine schlitzartige Öffnung (40) enthält, durch die eine chirurgische Zange (Klemme) einsetzbar ist.

4. Instrument nach Anspruch 1, bei dem die konische Spitzenfläche (33) und die kegelstumpfförmigen Flächen (35, 35', 35", 35"') zur Längsachse des Ladekegels (6) geneigt sind, wobei die Neigungswinkel jeweils von einer Fläche zur nächsten abnehmen.

5. Instrument nach Anspruch 4, bei dem der Ladekegel (6) vier kegelstumpfförmige Flächen aufweist und die Neigungswinkel der konischen Spitzenfläche (33) und der kegelstumpfförmigen Flächen (35, 35', 35", 35''') zur Längsachse des Ladekegels (6) jeweils 15°, 10°, 8°, 5° bzw. 3° betragen.

6. Instrument nach Anspruch 1, bei dem der Übergangsbereich zwischen dem konischen Teil (32) und dem rohrförmigen Verbindungsteil (31) des Ladekegels (6) einen ringförmigen Sitz (36) mit einem Boden (37) und einer Außenwand (38) aufweist derart, dass bei in die Öffnung (10) im distalen Ende (9) des Tubus (3) eingesetztem Ladekegel (6) die Außenwand (38) des ringförmigen Sitzes (36) den Endteil des distalen Endes (9) abdeckt.

7. Instrument nach einem der Ansprüche 1 bis 6, bei dem das distale Ende (9) des Tubus (3) zur Längsachse des Tubus (3) geneigt verläuft.

8. Instrument nach Anspruch 7, bei dem das distale Ende (9) mit 18° abwärts geneigt ist.

9. Instrument nach einem der Ansprüche 1 bis 8, bei dem der Abzug (4) an den Griff (2) und an das verschiebbare Betätigungsglied (5) mit einer Rasteinrichtung (14,19; 24, 29) angelenkt ist.

10. Instrument nach Anspruch 9, bei dem die Rasteinrichtung zylindrische Ansätze (14, 29) aufweist, die im Querschnitt kolbenförmig und in komplementäre Sitzmulden (19, 24) einrastbar sind.

11. Instrument nach Anspruch 10, bei dem die Sitzmulde (19) seitlich durch ein Paar Seitenwandflächen (21) teilweise oder vollständig geschlossen ist.

12. Instrument nach Anspruch 10, bei dem ein Zahn (25) mit einer bogenförmigen Kante mittig auf der Innenfläche der Sitzmulde (24) angeordnet ist und der Ansatz (29) in seiner Mitte eine Kerbe (30) enthält, wobei der Zahn (25) in die Kerbe (30) eingreifen soll.

13. Instrument nach einem der Ansprüche 1 bis 12, bei dem der Abzug (4) eine Zunge (22) aufweist, die elastisch auf den Griff (2) einwirkt.

14. Instrument nach einem der Ansprüche 1 bis 13, bei dem das verschiebbare Betätigungsglied (5) einen Hauptteil (26) mit einer halbzylindrischen Innenfläche aufweist, der an einem Ende zu einem Ring (27) ausläuft, der auf das distale Ende (9) des Tubus (3) aufschiebbar ist.

15. Instrument nach einem der Ansprüche 1 bis 14, das aus einem schlagfesten Polystyrol oder aus Polycarbonat gefertigt ist.

16. Instrument nach einem der Ansprüche 1 bis 15, das ein Einweginstrument ist.

17. Ladekegel (6) für ein Instrument zum Abbinden von Hämorrhoiden, der einen allgemein konischen Teil (32) aufweist, der an seiner Basis über einen ringförmigen Sitz (36) mit einem rohrförmigen Verbindungsteil (31) eines Durchmessers verbunden ist, der kleiner ist als der der Basis des allgemein konischen Teils, wobei die Außenfläche des allgemein konischen Teils eine konische Spitzenfläche (33) und eine erste kegelstumpfförmige Fläche (35) aufweist, die mit der konischen Spitzenfläche über einen zylindrischen Absatz (34) verbunden ist, **dadurch gekennzeichnet, dass** die Außenfläche des allgemein konischen Teils weiterhin mindestens eine weitere kegelstumpfförmige Fläche (35', 35", 35"') aufweist, die durch weitere zugehörige zylindrische Absätze (34', 34", 34"') angeschlossen ist.

18. Ladekegel nach Anspruch 17, bei dem die konische Spitzenfläche (33) und die kegelstumpfförmigen Flächen (35, 35', 35", 35"') eine Neigung zur Längsachse des Ladekegels (6) aufweisen, die von einer Fläche zur Nächsten jeweils abnimmt.

19. Ladekegel nach Anspruch 18, bei dem der Ladekegel (6) vier kegelstumpfförmige Flächen aufweist und die Neigungswinkel der konischen Spitzenfläche (33) und der kegelstumpfförmigen Flächen (35, 35', 35", 35"') zur Längsachse des Ladekegels (6) 15°, 10°, 8°, 5° bzw.3° betragen.

20. Ladekegel nach Anspruch 17, bei dem der Übergangsbereich zwischen dem allgemein konischen Bereich (32) und dem rohrförmigen Verbindungsteil (31) des Ladekegels (6) eine ringförmige Sitzfläche (36) mit einem Boden (37) und einer Außenwand (38) aufweist derart, dass bei in die Öffnung (10) im distalen Ende (9) des Tubus (3) eingesetztem Ladekegel (6) die Außenwand (38) des ringförmigen Sitzes (36) den Endteil des distalen Endes (9) abdeckt.

21. Teilesatz mit einem Instrument zum Abbinden von Hämorrhoiden nach einem der Ansprüche 1 bis 16, einem oder mehreren Ladekegeln (6) nach einem der Ansprüche 1 bis 16 und einem oder mehreren elastischen Ringen (7).

22. Teilesatz nach Anspruch 18, bei dem die elastischen Ringe (7) aus Naturgummi hergestellt sind.

## Revendications

1. Dispositif destiné à appliquer des ligatures aux hémorroïdes, comprenant un cylindre (3) fourni avec une poignée (2) et possédant une extrémité distale (9) qui peut recevoir des anneaux élastiques (7), un vérin coulissant (5), actionné par un déclencheur (4), étant disposé sur la surface externe du cylindre (3), le vérin coulissant (5) étant mobile entre une position rétractée et une position avancée afin que l'on puisse désengager l'anneau élastique (7) de l'extrémité distale (9) du cylindre (3) sous commande, ledit dispositif comprenant en plus un cône de chargement (6) pour les anneaux élastiques (7), le cône de chargement (6) pouvant être adapté de façon amovible dans l'ouverture (10) de l'extrémité distale (9) du cylindre (3) et comprenant une partie généralement conique (32) et une partie tubulaire de connexion (31) que l'on a conçue pour être adaptée dans l'ouverture (10) de l'extrémité distale (9) du cylindre (3), la surface externe de la partie conique (32) comprenant une surface d'embout conique (33) et une pluralité de surfaces tronconiques (35, 35', 35'', 35'''), la surface d'embout conique et les surfaces tronconiques étant bien séparées par des échelons cylindriques respectifs (34, 34', 34'', 34''').

2. Dispositif selon la revendication 1 dans lequel le cylindre (3) est basiquement constitué par un corps tubulaire (8) ayant une ouverture à décharge sous aspiration (11) disposée à l'extrémité du cylindre (3) éloignée de l'extrémité distale (9) et dans laquelle la poignée (2) comprend un conduit d'aspiration (12), le corps tubulaire (8) et le conduit (12) étant en communication fluide.

3. Dispositif selon la revendication 1 ou 2, dans lequel le cylindre (3) a une ouverture en encoche (40) dans sa partie supérieure à la proximité de l'extrémité distale (9), pour l'introduction d'un forceps chirurgical.

4. Dispositif selon la revendication 1, dans lequel la surface d'embout conique (33) et les surfaces tronconiques (35, 35', 35'', 35''') ont des inclinaisons respectives par rapport à l'axe longitudinal du cône de chargement (6) qui décroissent dans l'ordre d'une surface à la suivante.

5. Dispositif selon la revendication 4, dans lequel le cône de chargement (6) comprend quatre surfaces tronconiques et dans lequel les inclinaisons de la surface d'embout conique (33) et des surfaces tronconiques (35, 35', 35'', 35''') par rapport à l'axe longitudinal du cône de chargement (6) sont de 15°, 10°, 8°, 5° et 3°, respectivement.

6. Dispositif selon la revendication 1, dans lequel la région de jonction entre la partie conique (32) et la partie tubulaire de connexion (31) du cône de chargement (6) comprend une embase annulaire (36) ayant une base (37) et une paroi externe (38) afin que, lorsque l'on adapte le .cône de chargement (6) dans l'ouverture (10) dans l'extrémité distale (9) du cylindre (3), la paroi externe (38) de l'embase annulaire (36) recouvre la partie terminale de l'extrémité distale (9).

7. Dispositif selon l'une quelconque des revendications 1 à 6, dans lequel l'extrémité distale (9) du cylindre (3) est inclinée vers l'axe longitudinal du cylindre (3).

8. Dispositif selon la revendication 7, dans lequel l'extrémité distale (9) est inclinée de 18° vers le bas.

9. Dispositif selon l'une quelconque des revendications 1 à 8, dans lequel le déclencheur (4) est articulé à la poignée (2) et au vérin coulissant (5) par des moyens de fermoir (14, 19 ;24, 29).

10. Dispositif selon la revendication 9, dans lequel les moyens de fermoir comprennent des appendices cylindriques (14, 29) ayant des sections croisées à boutons et que l'on peut engager en fermoir dans des embases complémentaires (19, 24).

11. Dispositif selon la revendication 10, dans lequel l'embase (19) est partiellement ou complètement fermée latéralement par une paire de parois latérales (21).

12. Dispositif selon la revendication 10, dans lequel on dispose une dent (25) avec un bord arqué dans une position centrale sur la surface interne de l'embase (24), et dans laquelle l'appendice (29) a un cran (30) dans sa partie centrale, la dent (25) étant supposée s'engager dans le cran (30).

13. Dispositif selon l'une quelconque des revendications 1 à 12, dans lequel le déclencheur (4) comprend une languette (22) qui agit sur la poignée (2) comme un ressort.

14. Dispositif selon l'une quelconque des revendications 1 à 13, dans lequel le vérin coulissant (5) comprend un corps (26) ayant une surface interne semi-cylindrique et se terminant à une extrémité en un anneau (27) qui est supposé s'engager de façon coulissante sur l'extrémité distale (9) du cylindre (3).

15. Dispositif selon l'une quelconque des revendications 1 à 14, dans lequel le dispositif est fait de polystyrène ou de polycarbonate résistants aux chocs.

16. Dispositif selon l'une quelconque des revendications 1 à 15, dans lequel le dispositif est jetable.

17. Cône de chargement pour un dispositif destiné à appliquer des ligatures aux hémorroïdes, ledit cône de chargement ayant une partie généralement conique (32) liée à sa base par une embase annulaire (36) à une partie tubulaire de connexion (31) d'un diamètre inférieur au diamètre de la base de la partie généralement conique, la surface externe de ladite partie généralement conique comprenant une surface d'embout conique (33) et une première surface tronconique (35), connecté à la surface d'embout conique par une surface cylindrique à échelon (34), **caractérisé en ce que** la surface externe de partie généralement conique comprend en plus au moins une autre surface tronconique (35', 35'', 35'''), connectée respectivement par d'autres surfaces cylindriques à échelon (34' , 34'', 34''').

18. Cône de chargement selon la revendication 17, dans lequel la surface d'embout conique (33) et les surfaces tronconiques (35, 35', 35'', 35'''), ont des inclinaisons respectives par rapport à l'axe longitudinal du cône de chargement (6) qui décroissent dans l'ordre d'une surface à la suivante.

19. Cône de chargement selon la revendication 18, dans lequel le cône de chargement (6) comprend quatre surfaces tronconiques et dans lequel les inclinaisons de la surface d'embout conique (33) et des surfaces tronconiques (35, 35' , 35'', 35''') par rapport à l'axe longitudinal du cône de chargement (6) sont de 15°, 10°, 8°, 5° et 3°, respectivement.

20. Cône de chargement selon la revendication 17, dans lequel la région de jonction entre la partie généralement conique (32) et la partie tubulaire de connexion (31) du cône de chargement (6) comprend une embase annulaire (36) ayant une base (37) et une paroi externe (38) afin que, lorsque l'on adapte le cône de chargement (6) dans l'ouverture (10) dans l'extrémité distale (9) du cylindre (3), la paroi externe (38) de l'embase annulaire (36) recouvre la partie terminale de l'extrémité distale (9).

21. Kit comprenant un dispositif destiné à appliquer des ligatures aux hémorroïdes selon l'une quelconque des revendications 1 à 16, un cône de chargement (6) ou plus selon l'une quelconque des revendications 7 à 20, et un anneau élastique (7) ou plus.

22. Kit selon la revendication 18, dans lequel les anneaux élastiques (7) sont faits de caoutchouc naturel.
